(19) Europäisches Patentamt
European Patent Office
Office européen des brevets

(11) Veröffentlichungsnummer: **0 256 396**
**A1**

(12) # EUROPÄISCHE PATENTANMELDUNG

(21) Anmeldenummer: **87111105.0**

(22) Anmeldetag: **31.07.87**

(51) Int. Cl.⁴: **C07D 487/04**, A01N 43/90, //(C07D487/04,249:00,239:00)

(30) Priorität: **13.08.86 DE 3627411**

(43) Veröffentlichungstag der Anmeldung: **24.02.88 Patentblatt 88/08**

(84) Benannte Vertragsstaaten: **AT BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Jelich, Klaus, Dr.**
**Pahlkestrasse 5**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R, Dr.**
**Im Waldwinkel 110**
**D-5060 Bergisch Gladbach 2(DE)**
Erfinder: **Strang, Harry, Dr.**
**Untendorfstrasse 6A**
**D-4000 Düsseldorf 31(DE)**

(54) **Triazolo-pyrimidin-2-sulfonamide.**

(57) Die Erfindung betrifft neue Triazolo-pyrimidin-2-sulfonamide der Formel (I)

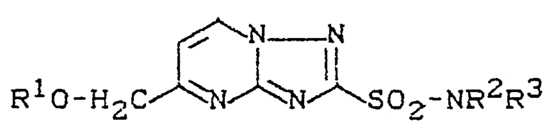

(I)

in welcher
$R^1$ für Alkyl steht,
$R^2$ für Wasserstoff, Alkyl, Alkylcarbonyl, Alkoxycarbonyl, Alkylsulfonyl, Alkenyl, Alkinyl oder für gegebenenfalls substituiertes Aralkyl steht und
$R^3$ für gegebenenfalls substituiertes Aryl steht,
ein Verfahren und neue Zwischenprodukte zu ihrer Herstellung und ihre Verwendung als Herbizide.

EP 0 256 396 A1

## Triazolo-pyrimidin-2-sulfonamide

Die Erfindung betrifft neue Triazolo-pyrimidin-2-sulfonamide, ein Verfahren und neue Zwischenprodukte zu ihrer Herstellung und ihre Verwendung als Herbizide.

Es ist bereits bekannt, daß bestimme Triazolo-pyrimidin-2-sulfonamid-Derivate, wie z. B. 2,6-Dimethyl-N-(2-methoxycarbonylphenyl)-1,2,4-triazolo-[1,5,-a]-pyramidin-2-sulfonamid herbizide Eigenschaften aufweisen (vergl. z. B. EP-A 142 152). Die Wirkung dieser Verbindung ist gut, jedoch werden bei geringen Aufwandmengen einige Unkräuter nicht immer voll erfaßt, außerdem ist die Selektivität nicht immer zufriedenstellend.

Es werden nun neue Triazolo-pyrimidin-2-sulfonamide der Formel (I) gefunden,

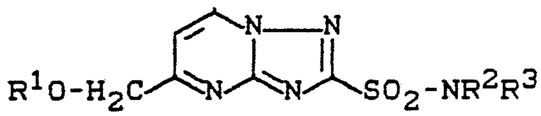

(I)

in welcher

$R^1$ für Alkyl steht,

$R^2$ für Wasserstoff, Alkyl, Alkylcarbonyl, Alkoxycarbonyl, Alkylsulfonyl, Alkenyl, Alkinyl oder für gegebenenfalls substituiertes Aralkyl steht und

$R^3$ für gegebenenfalls substituiertes Aryl steht.

Weiterhin wurde gefunden, daß man die neuen Triazolo-pyrimidin-2-sulfonamide der Formel (I) erhält, wenn man Triazolo-pyrimidin-sulfonsäurechloride der Formel (II)

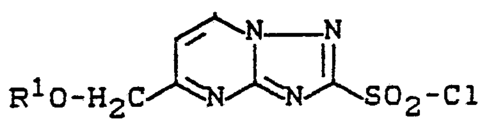

(II)

in welcher

$R^1$ die oben angegebene Bedeutung hat,

mit Aminen der Formel (III)

$R^3NH_2$ (III)

in welcher

$R^3$ die oben angegebene Bedeutung hat,

gegebenenfalls in Gegenwart von Säureakzeptoren, gegebenenfalls in Gegenwart von Katalysatoren und gegebenenfalls in Gegenwart von Verdünnungsmitteln umsetzt und gegebenenfalls anschließend die dabei entstehenden Triazolo-pyrimidin-2-sulfonamide der Formel (Ia)

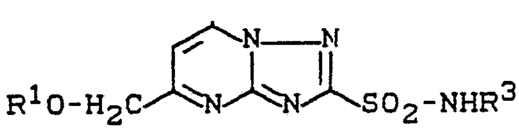

(Ia)

in welcher

$R^1$ und $R^3$ oben angegebenen Bedeutung haben,

mit Verbindungen der Formel (IV)

$R^4W$ (IV)

in welcher

$R^4$ die gleiche Bedeutung wie $R^2$ hat, jedoch nicht für Wasserstoff steht und

W eine für nucleophile Abgangsgruppe steht,

gegebenenfalls in Gegenwart von Säureakzeptoren und gegebenenfalls in Gegenwart von Verdünnungsmitteln umsetzt.

Schließlich wurde gefunden, daß sich die neuen Triazolo-pyrimidin-2-sulfonamide der Formel (I) durch eine hervorragende herbizide Wirkung auszeichnen. Überraschenderweise besitzen die erfindungsgemäßen Triazolo-pyrimidin-2-sulfonamide eine wesentlich bessere herbizide Wirksamkeit als die Verbindung 2,6-Dimethyl-N-(2-methoxycarbonylphenyl)-1,2,4-triazolo-[1,5-a]-pyrimidin-2-sulfonamid, welcher ein konstitutionell ähnlicher Wirkstoff gleicher Wirkungsrichtung ist.

Bei den aliphatischen Substituenten ist die Kohlenstoffkette in den Definitionen jeweils geradkettig oder verzweigt. Halogen steht jeweils für Fluor, Chlor, Brom und Iod.

Die erfindungsgemäßen Triazolo-pyrimidin-2-sulfonamide sind durch die Formel (I) allgemein definiert. Bevorzugt sind diejenigen Verbindungen der Formel (I), in denen

$R^1$ für Alkyl mit 1 bis 6 Kohlenstoffatomen steht,

$R^2$ für Wasserstoff, für Alkyl, Alkylcarbonyl, Alkoxycarbonyl und Alkylsulfonyl mit jeweils 1 bis 6 Kohlenstoffatomen im Alkylteil, für Alkenyl und Alkinyl mit jeweils 3 bis 8 Kohlenstoffatomen oder für gegebenenfalls einfach oder mehrfach, gleich oder verschieden im Arylteil substituiertes Aralkyl mit 6 bis 10 Kohlenstoffatomen im Arylteil und 1 bis 4 Kohlenstoffatomen im Alkylteil steht, wobei als Arylsubstituenten in Frage kommen:

Halogen, wie Fluor, Chlor, Brom oder Iod, Cyano, Nitro, Hydroxy, $C_1$-$C_6$-Alkyl, $C_1$-$C_6$-Alkoxy, $C_1$-$C_6$-Alkylthio, Halogen-$C_1$-$C_6$-alkyl, Halogen-$C_1$-$C_6$-alkoxy oder Halogen-$C_1$-$C_6$-alkylthio und

$R^3$ für gegebenenfalls einfach bis mehrfach, gleich oder verschieden substituiertes Aryl mit 6 bis 10 Kohlenstoffatomen steht, wobei als Arylsubstituenten in Frage kommen:

Halogen, wie Fluor, Chlor, Brom oder Iod; Nitro; Cyano; Hydroxy; $C_1$-$C_6$-Alkyl; $C_1$-$C_6$-Alkoxy, $C_1$-$C_6$-Alkylthio; Halogen-$C_1$-$C_6$-alkyl; Halogen-$C_1$-$C_6$-alkoxy, Halogen-$C_1$-$C_6$-alkylthio; $C_1$-$C_6$-Alkylcarbonyl; $C_1$-$C_6$-Alkylsulfinyl; $C_1$-$C_6$-Alkylsulfonyl; Halogen-$C_1$-$C_6$-alkylsulfinyl; Halogen-$C_1$-$C_6$-alkylsulfonyl; Phenyl; Phenoxy; Phenylcarbonyl; Hydroxycarbonyl, $C_1$-$C_6$-Alkoxycarbonyl; $C_3$-$C_6$-Alkenyloxycarbonyl; $C_1$-$C_4$-Alkoxy-$C_1$-$C_6$-alkoxycarbonyl; Hydroximino oder $C_1$-$C_6$-Alkoximino.

Besonders bevorzugt sind die Verbindungen der Formel (I), in welcher

$R^1$ für Alkyl mit 1 bis 4 Kohlenstoffatomen steht,

$R^2$ für Wasserstoff, $C_1$-$C_4$-Alkyl, $C_1$-$C_4$-Alkylcarbonyl, $C_1$-$C_4$-Alkoxycarbonyl, $C_1$-$C_4$-Alkylsulfonyl, $C_3$-$C_6$-Alkenyl, $C_3$-$C_6$-Alkinyl und für gegebenenfalls einfach bis dreifach, gleich oder verschieden im Phenylteil substituiertes Phenyl-$C_1$-$C_4$-alkyl steht, wobei als Phenylsubstituenten in Frage kommen:

Fuor, Chlor, Brom, Iod, Hydroxy, Cyano, Nitro, Methyl, Ethyl, n-Propyl, i-Propyl, n-Butyl, i-Butyl, sec.-Butyl, tert.-Butyl, Methoxy, Ethoxy, n-Propoxy, i-Propoxy, n-Butoxy, i-Butoxy, sec.-Butoxy, tert.-Butoxy, Methylthio, Ethylthio, n-Propylthio, i-Propylthio, n-Butylthio, i-Butylthio, sec.-Butylthio, tert.-Butylthio, Trifluormethyl, Trifluormethoxy oder Trifluormethylthio und

$R^3$ für gegebenenfalls einfach bis dreifach, gleich oder verschieden substituiertes Phenyl steht, wobei als Phenylsubstituenten in Frage kommen:

Fluor, Chlor, Brom, Iod, Nitro, Cyano, Hydroxy, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy, Halogen-$C_1$-$C_4$-alkylthio, $C_1$-$C_4$-Alkylcarbonyl, $C_1$-$C_4$-Alkylsulfinyl, $C_1$-$C_4$-Alkylsulfonyl, Halogen-$C_1$-$C_4$-alkylsulfinyl, Halogen-$C_1$-$C_4$-alkylsulfonyl, Phenyl, Phenoxy, Phenylcarbonyl, Hydroxycarbonyl, $C_1$-$C_4$-Alkoxycarbonyl, $C_3$-$C_6$-Alkenyloxycarbonyl, $C_1$-$C_2$-Alkoxy-$C_1$-$C_4$-alkoxycarbonyl, Hydroximino oder $C_1$-$C_4$-Alkoximino.

Ganz besonders bevorzugt sind die Verbindungen der Formel (I), in welcher,

$R^1$ für Methyl, Ethyl, n-Propyl, i-Propyl, n-Butyl, i-Butyl oder sec.-Butyl steht,

$R^2$ für Wasserstoff, Methyl, Ethyl, Methylcarbonyl, Ethylcarbonyl, Methoxycarbonyl, Ethoxycarbonyl, Methylsulfonyl, Ethylsulfonyl, 2-Propen-1-yl, 2-Propin-2-yl und für gegebenenfalls einfach bis dreifach, gleich oder verschieden im Phenylteil substituiertes Benzyl steht, wobei als Phenylsubstituenten in Frage kommen:

Fluor, Chlor, Brom, Iod, Nitro, Hydroxy, Methyl, Methoxy, Methylthio, Trifluormethyl, Trifluormethoxy oder Trifluormethylthio und

$R^3$ für gegebenenfalls einfach bis dreifach, gleich oder verschieden substituiertes Phenyl steht, wobei als Phenylsubstituenten in Frage kommen:

Fluor, Chlor, Brom, Iod, Nitro, Hydroxy, Methyl, Methoxy, Methylthio, Trifluormethyl, Trifluormethoxy, Trifluormethylthio, Methylcarbonyl, Ethylcarbonyl, Methylsulfinyl, Methylsulfonyl, Trifluormethylsulfinyl, Trifluormethylsulfonyl, Phenyl, Phenoxy, Phenylcarbonyl, Hydroxycarbonyl, Methoxycarbonyl, Ethoxycarbonyl, 2-Propen-1-yloxycarbonyl, Methoxy-methoxycarbonyl, Ethoxy-methoxycarbonyl, Methoxy-ethoxycarbonyl, Ethoxy-ethoxycarbonyl, Hydroximino, Methoximino, Ethoximino oder n-Propoximino.

Verwendet man 5-Methoxymethyl-1,2,4-triazol-[1,5-a]-pyrimidin-2-sulfonylchlorid und 2-Chlor-6-methylanilin als Ausgangsstoffe, so kann der Verlauf des erfindungsgemäßen Verfahrens durch das folgende Formelschema wiedergegeben werden:

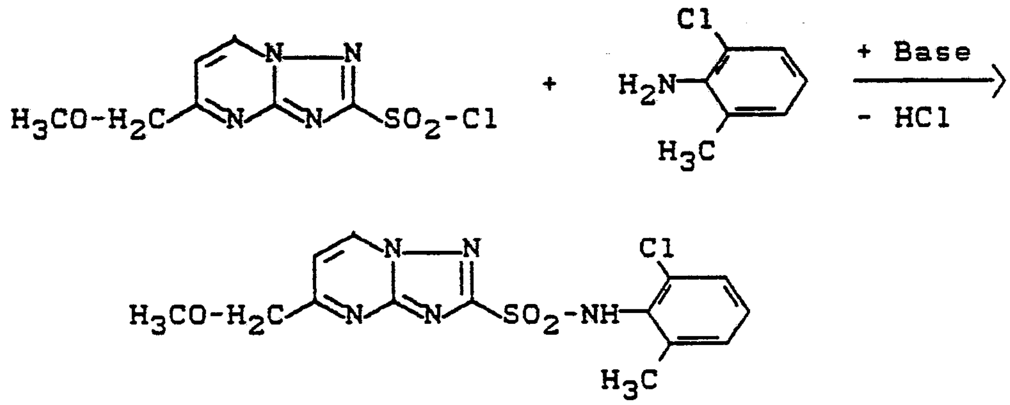


Verwendet man 5-Methoxymethyl-1,2-4-triazol-[1,5-a]-pyrimidin-2-sulfonylchlorid, 2-Chlor-6-methylanilin und Methyliodid als Ausgangsstoffe so kann der Verlauf des erfindungsgemäßen Verfahrens durch das folgende Formelschema wiedergegeben werden:

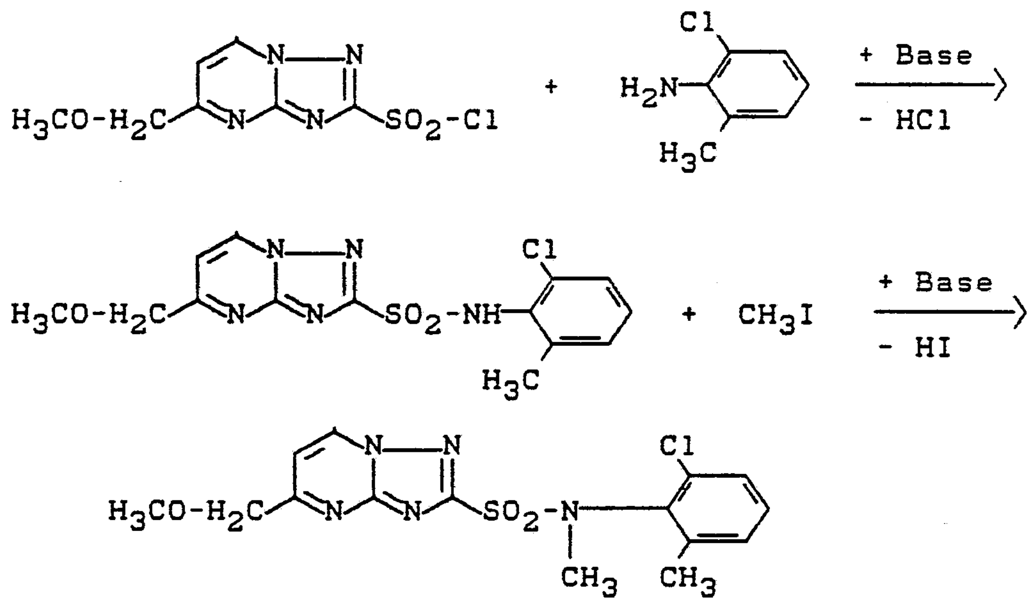


Die bei dem erfindungsgemäßen Verfahren als Ausgangsstoffe zu verwendenden Triazolo-pyrimidin-sulfonsäurechloride sind durch die Formel (II) allgemein definiert. In dieser Formel (II) steht $R^1$ vorzugsweise für diejenigen Reste, die bereits im Zusammenhang mit der Beschreibung der erfindungsgemäßen Stoffe der Formel (I) vorzugsweise für diesen Substituenten genannt wurden.

Als Beispiele für die Triazolo-pyrimidin-sulfonsäurechloride der Formel (II) seien genannt: 5-Methoxymethyl-, 5-Ethoxymethyl-, 5-n-Propoxymethyl-, 5-i-Propoxymethyl-, 5-n-Butoxymethyl-, 5-i-Butoxymethly-und 5-sec.-Butoxymethyl-1,2,4-triazol-[1,5-a]-pyrimidin-sulfonylchlorid.

Die Verbindungen der Formel (II) sind neu und Teil der vorliegenden Erfindung. Sie lassen sich jedoch in einfacher Weise nach bekannten Methoden herstellen.

So erhält man die neuen Verbindungen der Formel (II)

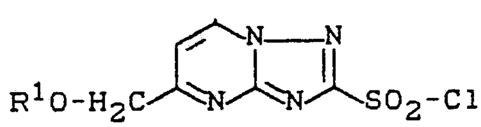

(II)

in welcher

$R^1$ für Alkyl steht,

wenn man Benzylsulfide der Formel (V)

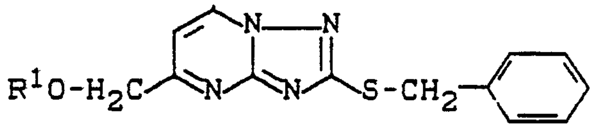

(V)

in welcher
R[1] die oben angegebene Bedeutung hat,
mit einem Chlorierungsmittel, wie z. B. elementarem Chlor oder Natriumhypochlorit, in Gegenwart von wäßrigen Säuren wie z. B. wäßriger Salzsäure oder Essigsäure und gegebenenfalls in Gegenwart von wasserunlöslichen organischen Verdünnungsmitteln wie z. B. Methylenchlorid oder Chloroform bei Temperaturen zwischen -20°C und +25°C umsetzt.

Die Benzylsulfide der Formel (V) sind neu. Man erhält die Verbindungen der Formel (V)

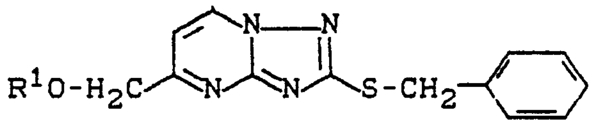

(V)

in welcher
R[1] für Alkyl steht,
wenn man Verbindungen der Formel (VI)

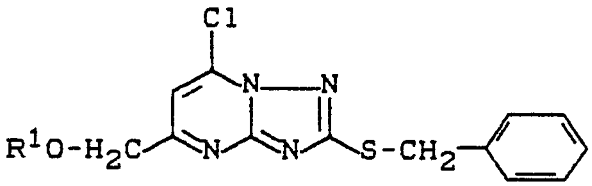

(VI)

in welcher
R[1] die oben angegebene Bedeutung hat,
in Gegenwart von Reduktionsmitteln, wie z.B. Zinkstaub und Kupfersulfat, in Gegenwart von Säuren, wie z. B. Eisessig, und in Gegenwart von Verdünnungsmitteln, wie z. B. Methanol und Tetrahydrofuran, bei Temperaturen zwischen 0°C und 30°C umsetzt.

Die Verbindungen der Formel (VI) sind neu. Man erhält die Verbindungen der Formel (VI)

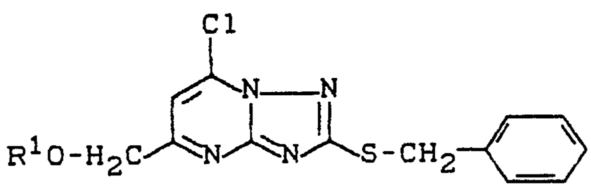

(VI)

in welcher
R[1] für Alkyl steht,
wenn man Hydroxy-Derivate der Formel (VII)

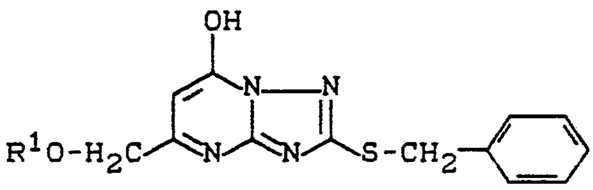

(VII)

in welcher

$R^1$ die oben angegebene Bedeutung hat,

mit Phosphoroxychlorid, gegebenenfalls in Gegenwart von Verdünnungsmitteln, wie z. B. Acetonitril, bei Temperaturen von 60°C bis 120°C umsetzt.

Die Hydroxy-Derivate der Formel (VII) sind neu. Man erhält die Verbindungen der Formel (VII)

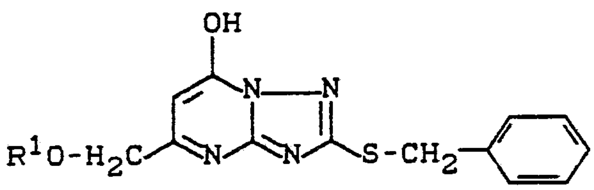

(VII)

in welcher

$R^1$ für Alkyl steht,

wenn man 5-Amino-3-benzylthio-1,2,4-triazol der Formel (VIII)

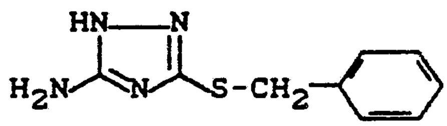

(VIII)

mit Alkoxyacetessigsäureestern der Formel (IX)

$$R^1O-CH_2-\overset{O}{\overset{\|}{C}}-CH_2-COOR^5 \quad (IX)$$

in welcher

$R^1$ die oben angegebenen Bedeutungen hat und

$R^5$ für $C_1$-$C_4$-Alkyl steht,

in Gegenwart von Säuren, wie z. B. Eisessig, bei Temperaturen von 80°C bis 140°C umsetzt.

Die Verbindungen der Formeln (VIII) und (IX) sind bekannt (vergl. z. B. J. Het. Chem 12, 1887 (1975) und DE-OS 22 44 012).

Die Verbindungen der Formeln (V), (VI) und (VII) lassen sich in einer gemeinsamen Formel (X) zusammenfassen:

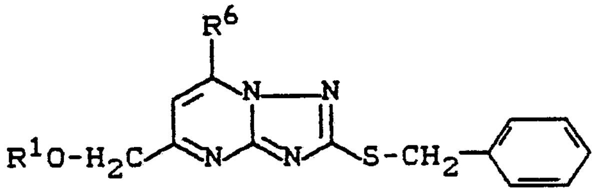

(X)

in welcher

$R^1$ für Alkyl, vorzugsweise für $C_1$-$C_6$-Alkyl, insbesondere für $C_1$-$C_4$-Alkyl steht und
$R^6$ für Wasserstoff, Chlor oder Hydroxy steht.

Als Beispiele für die neuen Verbindungen der Formeln (V), (VI) und (VII) seien genannt:

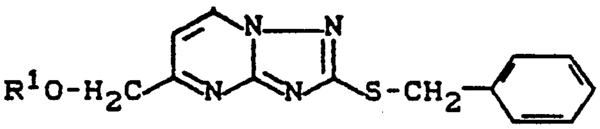

(V)

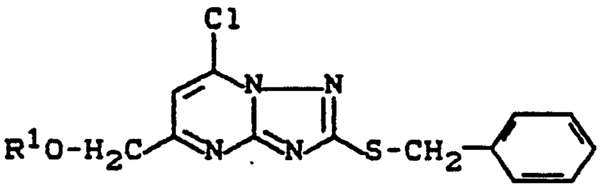

(VI)

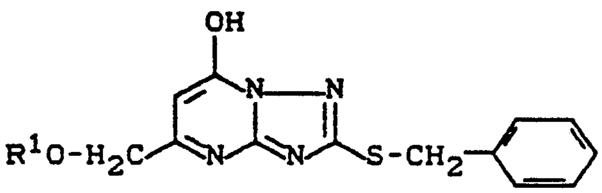

(VII)

Tabelle 1

| $R^1$ | $R^1$ |
|---|---|
| $CH_3$ | $n$-$C_4H_9$ |
| $C_2H_5$ | $i$-$C_4H_9$ |
| $n$-$C_3H_7$ | sec.-$C_4H_9$ |
| $i$-$C_3H_7$ | |

Die bei dem erfindungsgemäßen Verfahren außerdem als Ausgangsstoffe zu verwendenden Amine sind durch die Formel (III) allgemein definiert. In dieser Formel (III) steht $R^3$ vorzugsweise für diejenigen Reste, die bereits im Zusammenhang mit der Beschreibung der erfindungsgemäßen Stoffe der Formel (I) vorzugsweise für diesen Substituenten genannt wurden.

Die Amine der Formel (III) sind allgemein bekannte Verbindungen der organischen Chemie.

Als Beispiele für die Amine der Formel (III) seien genannt:

Tabelle 2

| $R^3$ | $R^3$ |
|---|---|
| 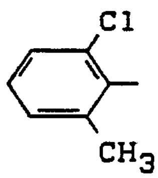 | 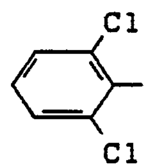 |
| 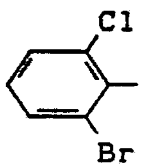 | 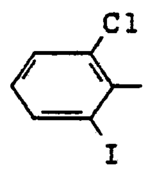 |
| 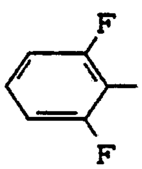 | 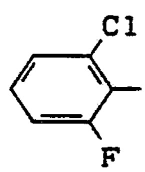 |
| 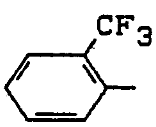 | 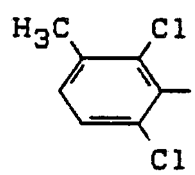 |

Tabelle 2 - Fortsetzung

| $R^3$ | $R^3$ |
| --- | --- |
| 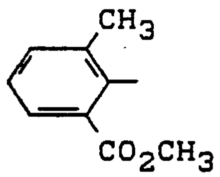 | 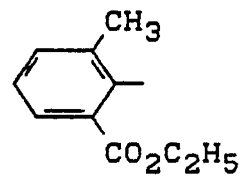 |
| 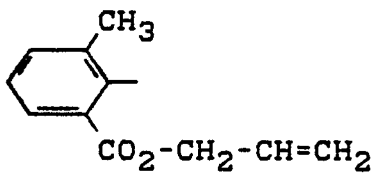 | 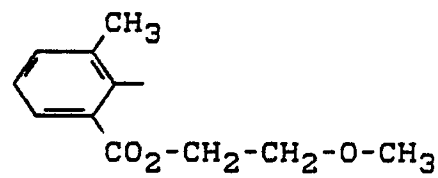 |
| 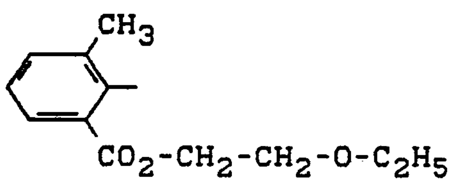 | 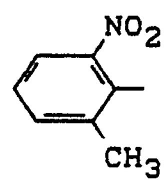 |
| 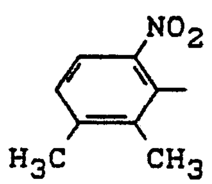 | |

Die bei dem erfindungsgemäßen Verfahren weiterhin als Ausgangsstoffe zu verwendenden Verbindungen sind durch die Formel (IV) allgemein definiert. In dieser Formel (IV) steht $R^4$ vorzugsweise für diejenigen Reste, die bereits im Zusammenhang mit der Beschreibung der erfindungsgemäßen Stoffe der Formel (I) vorzugsweise für den Substituenten $R^2$ genannt wurden, wobei jedoch $R^4$ nicht für Wasserstoff steht. W steht in Formel (IV) für eine nucleophile Abgangsgruppe, vorzugsweise für Chlorid, Bromid, Iodid, $-O-SO_2-OCH_3$, $O-SO_2CH_3$ und

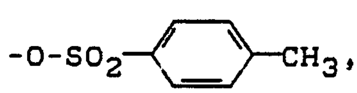

insbesondere für Chlorid.

Als Beispiele für die Verbindungen der Formel (IV) seien genannt:

$R^4W$ (IV)

W = Chlorid,

Tabelle 4

| $R^4$ | $R^4$ | $R^4$ |
|---|---|---|
| $-CH_3$ | $-C_2H_5$ | $-C_3H_7$-n |
| $-C_3H_7$-i | $-C_4H_9$-n | $-C_4H_9$-i |
| $-CO-CH_3$ | $-COC_2H_5$ | $-COOCH_3$ |
| $-COOC_2H_5$ | $-SO_2CH_3$ | $-SO_2C_2H_5$ |
| $-COOC_3H_7$-n | $-COOC_4H_9$-n | $-COC_3H_7$-n |
| $-COC_4H_9$-n | $-CH_2-CH=CH_2$ | $-CH_2-C \equiv CH$ |
| 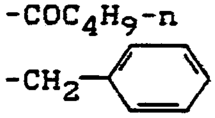 | | |

Das erfindungsgemäße Verfahren zur Herstellung der neuen Triazolo-pyrimidin-2-sulfonamide der Formel (Ia) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon, Hexamethylphosphorsäuretriamid und Pyridin.

Das erfindungsgemäße Verfahren zur Herstellung der Verbindungen der Formel (Ia) wird bevorzugt in Gegenwart von Säureakzeptoren durchgeführt. Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydride wie z. B. Natrium-und Kaliumhydrid, Alkalimetallalkyl-Verbindungen wie z. B. Butyllithium, Alkalimetallhydroxide wie z. B. Natrium-und Kaliumhydroxid, Erdalkalihydroxide wie z. B. Calciumhydroxid, Alkalicarbonate und-alkoholate wie Natrium-und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Dimethylaminopyridin, N-Methylmorpholin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Als Katalysatoren können bei dem erfindungsgemäßen Verfahren basische Katalysatoren verwendet werden. Vorzugsweise in Frage kommen: aliphatische, aromatische oder heterocyclische Amine wie Triethylamin, Trimethylamin, Dimethylaminopyridin, Dimethylanilin und N-Methylmorpholin.

Die Reaktionstemperaturen können bei dem erfindungsgemäßen Verfahren in einem größeren Bereich variiert werden. Im allgemeinen arbeitet man bei Temperaturen zwischen 0 °C und 160 °C , vorzugsweise bei Temperaturen zwischen 0 °C und 140 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe der Formeln (II) und (III) im allgemeinen in angenähert äquimolaren Mengen eingesetzt. Für den Fall, daß die Verbindung der Formel (III) eine geringe Reaktivität hat, ist es vorteilhafter zuerst das Amin der Formel (III) mit einer starken Base, wie z. B. Natriumhydrid oder Butyllithium bei Temperaturen zwischen -80 °C und 0 °C umzusetzen und das so erhaltene Metallderivat mit der Verbindung der Formel (II) weiter reagieren zu lassen. Im dem Fall setzt man auf 1 Mol der Verbindung der Formel (II) 2 bis 3 Mol Amin ein.

Die Reaktionen werden im allgemeinen in einem geeigneten Verdünnungsmittel in Gegenwart eines Säureakzeptors und gegebenenfalls in Gegenwart eines Katalysators durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Ist der Herstellung einer Verbindung der Formel (I) beabsichtigt, in der $R^2$ nicht Wasserstoff steht, so geht man so vor, daß man eine nach dem erfindungsgemäßen Verfahren hergestellte Verbindung der Formel (Ia) mit einer Verbindung der Formel (IV) umsetzt. Diese Umsetzung wird vorzugsweise in Gegenwart von geeigneten Verdünnungsmitteln durchgeführt. Als Verdünnungsmittel kommen dafür in Frage: Ether wie Diethyl-und Dibutylether, Glykoldimethylether und Di-glykoldimethylether, Tetrahydrofuran und Dioxan, Ketone wie Aceton, Methyl-ethyl-, Methyl-isopropyl-und Methylisobutyl-keton, Nitrile wie z. B. Acetonitril und Propionitril, Amide wie z. B. Dimethylformamid sowie Dimethylsulfoxid, Alkohole wie z. B. Methanol, Ethanol, Isopropanol und tert.-Butanol.

Als Säureakzeptoren für diese Umsetzungen kommen vorzugsweise in Frage: Alkalimetallhydroxide wie z. B. Natrium-und Kaliumhydroxid, Erdalkalihydroxide wie z. B. Calciumhydroxid, Alkalicarbonate und -alkoholate wie Natrium-und Kaliumcarbonat, Natrium-und Kaliummethylat bzw. -ethylat und Kalium-tert.-butylat, ferner aliphatische, aroma tische oder heterocyclische Amine, beispielsweise Triethylamin, Trimethylamin, Dimethylanilin, Dimethylbenzylamin, Pyridin und 4-Dimethylaminopyridin.

Die Reaktion für die Herstellung der Verbindungen der Formel (I) aus Verbindungen der Formel (Ia) werden im allgemeinen bei Temperaturen zwischen 0 °C und 120 °C, vorzugsweise bei Temperaturen zwischen 20 °C und 100 °C durchgeführt.

Zur Durchführung der Reaktion werden die Ausgangsstoffe der Formeln (Ia) und (IV) im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die erfindungsgemäßen Verbindungen der Formel (I) eignen sich besonders zur selektiven Bekämpfung mono-und dikotyler Unkräuter insbesondere im Nachauflaufverfahren in Kulturen wie z. B. Soja.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln - schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Säge mehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche, Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z. B. Ethyl-2-{[(4-chlor-6-methoxy-2-pyrimidyl)-aminocarbonyl]-aminosulfonyl}-benzoat, 4-Amino-6-(1,1-dimethylethyl)-3-methylthio-1,2,4-triazin-5(4H)-on, 4-Amino-3-methyl-6-phenyl-1,2,4-triazin-5(4H)-on, 5-(2-Chlor-4-trifluormethyl-phenoxy)-2-nitrobenzoesäure, 5-(2-Chlor-4-trifluormethyl-phenoxy)-N-methylsulfonyl-2-nitrobenzamid, (2-Ethoxy-1-methyl-2-oxo-ethyl)-5-(2-chlor-4-trifluormethyl-phenoxy)-2-nitro-benzoat, 2-{4-[(3-Chlor-5-trifluormethyl-2-pyridinyl)oxy]-phenoxy}-propionsäure, das R-Enantiomere des 2-{4-[(3,5-Dichlor-2-pyridinyl)oxy]-phenoxy}-propionsäure-(trimethylsilyl)-methylesters, 2,4-Dichlorphenoxyessigsäure, 2-(2,4-Dichlorphenoxy)-propionsäure, 4-Chlor-2-methyl-phenoxy-essigsäure, 2-(4-Chlor-2-methyl-phenoxy)-propionsäure, 2-[5-Methyl-5-(1-methyl-ethyl)-4-oxo-2-imidazolin-2-yl]-3-pyridincarbonsäure, 2-(1-Ethoxyamino-butyliden)-5-(2-ethylthiopropyl)-1,3-cyclohexandion, 2-[1-(Ethoxyimino)-butyl]-3-hydroxy-5-(tetrahydro-2H-thiopyran-3-yl)-2-cyclohexen-1-on, 2-(1-Alloxyaminobutyliden-4-methoxycarbonyl-5-5-dimethylcyclohexan-1,3-dion-Natriumsalz und 3-Isopropyl-2,1,3-benzothiadiazinon-(4)-2,2-dioxid. Einige Mischungen besitzen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen be reiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den eingearbeitet werden.

Die angewandte Wirkstoffmenge kann in eine, größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

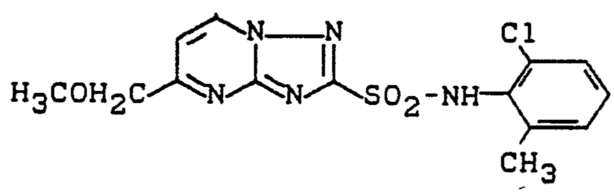

1,0 g (0,0038 Mol) 5-Methoxymethyl-1,2,4-triazol-[1,5-a]-pyrimidin-2-sulfonylchlorid werden zu einer Lösung von 0,3 g (0,0038 Mol) Pyridin, 0,05 g (0,00041 Mol) 4-Dimethylaminopyridin und 0,54 g (0,0038 Mol) 2-Chlor-6-methylanilin in 20 ml Dichlormethan gegeben und 24 Stunden bei 20 °C gerührt. Anschließend wird das Gemisch eingeengt und der Rückstand chromatographisch über Kieselgel gereinigt.

Man erhält 0,8 g (57 % der Theorie) 5-Methoxymethyl-N-(2-chlor-6-methyl-phenyl)-1,2,4-triazol-[1,5-a]-pyrimidin-2-sulfonamid als farbloser Feststoff vom Schmelzpunkt 196 °C.

Analog Beispiel 1 und den allgemeinen Angaben zur Herstellung können die nachfolgenden Verbindungen der Formel (I) hergestellt werden:

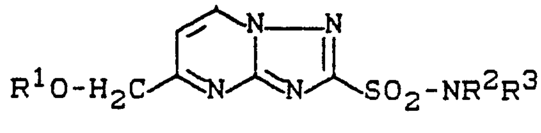

(I)

Tabelle 4

| Beisp.-Nr. | $R^1$ | $R^2$ | $R^3$ | Physikalische Daten |
|---|---|---|---|---|
| 2 | $CH_3$ | H | 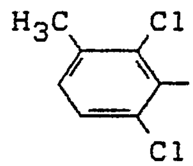 | Fp. 210 °C |
| 3 | $CH_3$ | H | 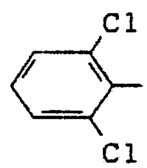 | Fp. 209 °C (Zers.) |
| 4 | $CH_3$ | H | 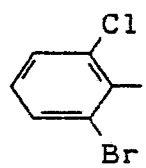 | |
| 5 | $CH_3$ | H | 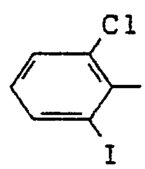 | |
| 6 | $CH_3$ | H | 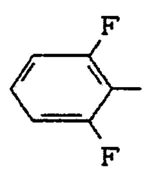 | Fp. 188° C |
| 7 | $CH_3$ | H | 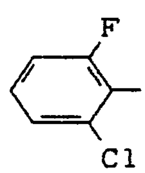 | |
| 8 | $CH_3$ | H | 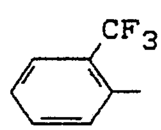 | |

Tabelle 4 - Fortsetzung

| Beisp.-Nr. | $R^1$ | $R^2$ | $R^3$ | Physikalische Daten |
|---|---|---|---|---|
| 9 | $C_2H_5$ | H | 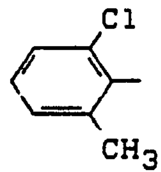 | |
| 10 | $C_2H_5$ | H | 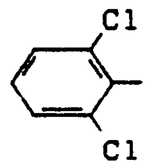 | |
| 11 | $C_2H_5$ | H | 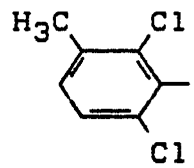 | |
| 12 | $CH_3$ | $CH_3$ | 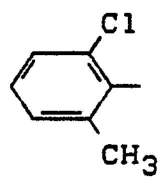 | |
| 13 | $CH_3$ | $-CH_2-$ 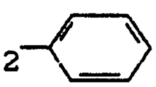 | 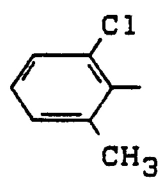 | |
| 14 | $CH_3$ | $-CH_2-CH=CH_2$ | 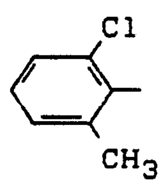 | |
| 15 | $CH_3$ | $-CH_2-C\equiv CH$ | 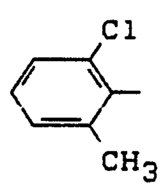 | |

Tabelle 4 - Fortsetzung

| Beisp.-Nr. | $R^1$ | $R^2$ | $R^3$ | Physikalische Daten |
|---|---|---|---|---|
| 16 | $CH_3$ | $-COCH_3$ | 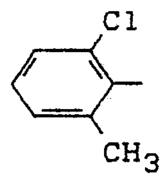 | |
| 17 | $CH_3$ | $-COOCH_3$ | 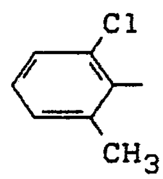 | |
| 18 | $CH_3$ | $-SO_2CH_3$ | 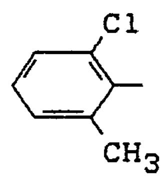 | |
| 19 | $CH(CH_3)_2$ | H | 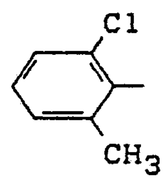 | |
| 20 | $CH(CH_3)_2$ | H | 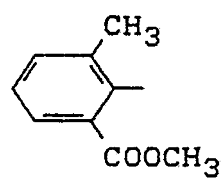 | |
| 21 | $CH(CH_3)_2$ | H | 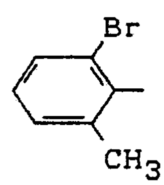 | |
| 22 | $CH_3$ | H | 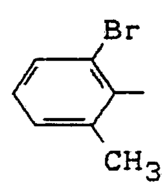 | |
| 23 | $CH_3$ | H | 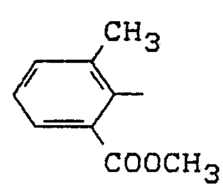 | Fp. 161°C |

Ausgangsprodukte der Formel (II)

Beispiel (II-1)

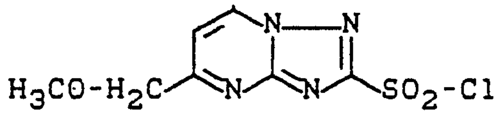

8,6 (0,030 Mol) 2-Benzylthio-5-methoxymethyl-1,2,4-triazol-[1,5-a]-pyrimidin werden in 50 ml Eisessig/Wasser 1 : 1 suspendiert und bei einer Temperatur von -5 °C wird 30 Minuten Chlorgas durchgeleitet. Das ausgefallene Produkt wird abgesaugt, mit Eisessig nachgewaschen und getrocknet.

Man erhält 5,3 g (67%) 5-Methoxymethyl-1,2,4-triazol-[1,5-a]-pyrimidin-2-sulfonylchlorid als beiges Pulver vom Schmelzpunkt 125 °C.

Analog Beispiel (II-1) können die übrigen Verbindungen der Formel (II) erhalten werden:

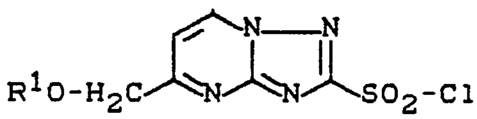

(II)

Tabelle 5

| Beispiel-Nr. | $R^1$ | Physikalische Daten |
|---|---|---|
| II-2 | $C_2H_5$ | |
| II-3 | $C_3H_7$-i | |

Ausgangsprodukte der Formel (V)

Beispiel (V-1)

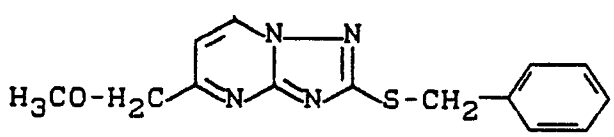

Ein Zink-Kupfer-Paar wird entsprechend J. Org. Chem 31, 626 (1966) hergestellt, indem man 10 g Kupfersulfat mit 150 g Zinkstaub in 200 ml Wasser 2 Stunden rührt. Das Produkt wird abgesaugt, mit Aceton gewaschen und über Nacht bei 100 °C im Vakuum getrocknet.

Zu 6,2 g (0,019 Mol) 2-Benzylthio-7-chlor-5-methoxymethyl-1,2,4-triazol-[1,5-a]-pyrimidin, 10 ml Methanol und 60 ml Tetrahydrofuran werden unter Außenkühlung bei 20 °C 2,32 g (0,039 Mol) Eisessig und anschließend 3,8 g des o. g. Zink-Kupfer-Paares gegeben, 2 Stunden gerührt und über Kieselgur gegossen. Das Filtrat wird eingeengt und der Rückstand chromatographisch über Kieselgel gereinigt.

Man erhält 5,3 g (96 % der Theorie) 2-Benzylthio-5-methoxymethyl-1,2,4-triazol-[1,5-a]-pyrimidin als gelber Feststoff vom Schmelzpunkt 92 °C.

Analog Beispiel (V-1) können die folgenden Verbindungen der Formel (V) erhalten werden:

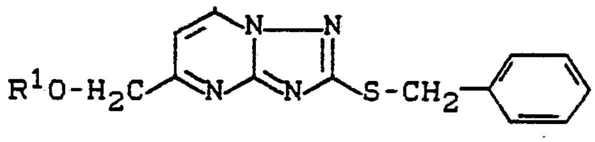

(V)

Tabelle 6

| Beispiel-Nr. | $R^1$ | Physikalische Daten |
|---|---|---|
| V-2 | $C_2H_5$ | |
| V-3 | $C_3H_7$-i | |

Ausgangsprodukte der Formel (VI)

Beispiel (VI-1)

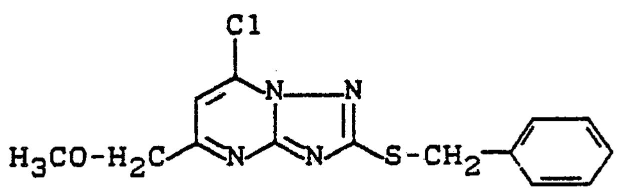

Eine Lösung von 22,8 g (0,075 Mol) 2-Benzylthio-5-methoxymethyl-7-hydroxy-1,2,4-triazol-[1,5-a]-pyrimidin und 35 g (0,228 Mol) Phosphoroxychlorid in 200 ml Acetonitril wird 4 Stunden unter Rückfluß erhitzt. Nach dem Abkühlen werden das Lösungsmittel und überschüssiges Phosphoroxychlorid entfernt. Der Rückstand wird in Methylenchlorid und Wasser aufgenommen, die organische Phase über Magnesiumsulfat getrocknet und im Vakuum eingeengt. Der Rück stand wird mit Ether als Laufmittel über eine Kieselgelsäule gereinigt.

Man erhält 19,0 g (78,5 % der Theorie) 2-Benzylthio-7-chlor-5-methoxymethyl-1,2,4-triazol-[1,5-a]-pyrimidin als farbloses Pulver vom Schmelzpunkt 79 °C.

Analog Beispiel (VI-1) können die folgenden Verbindungen der Formel (VI) erhalten werden:

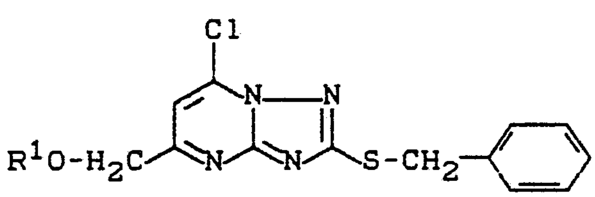

(VI)

Tabelle 7

| Beispiel-Nr. | $R^1$ | Physikalische Daten |
|---|---|---|
| VI-2 | $C_2H_5$ | |
| VI-3 | $C_3H_7$-i | |

Ausgangsprodukte der Formel (VII)

Beispiel (VII-1)

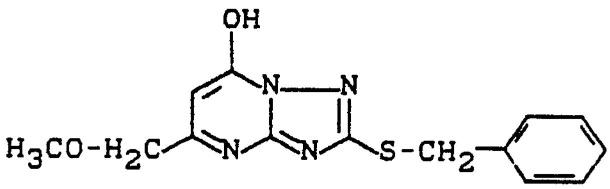

Ein Gemisch aus 20 g (0,97 Mol) 3-Amino-5-benzylthio-1,2,4-triazol und 14,2 g (0,097 Mol) 4-Methoxyacetessigsäuremethylester in 200 ml Eisessig werden 3 Stunden unter Rückfluß erhitzt. Nach dem Abkühlen wird das ausgefallene Produkt abgesaugt und mit eiskaltem Ethanol verrührt.

Man erhält 24 g (82 % der Theorie) 2-Benzylthio-7-hydroxy-5-methoxymethyl-1,2,4-triazol-[1,5-a]-pyrimidin vom Schmelzpunkt 213 °C.

Analog Beispiel (VII-1) können die folgenden Verbindungen der Formel (VII) erhalten werden:

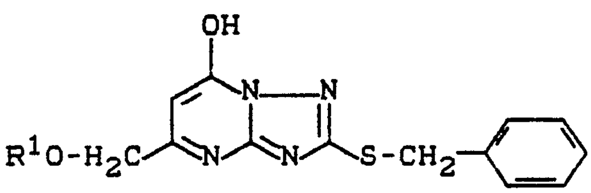

(VII)

Tabelle 8

| Beispiel-Nr. | $R^1$ | Physikalische Daten |
|---|---|---|
| VII-2 | $C_2H_5$ | |
| VII-3 | $C_3H_7$-i | |

Anwendungsbeispiele

In dem nachfolgenden Anwendungsbeispiel wird die nachstehend angegebene Verbindung als Vergleichsverbindung eingesetzt:

(A)

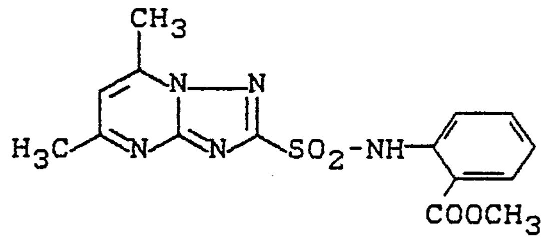

bekannt aus EP-A 142 152

Beispiel A

Post-emergence-Test Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit gegen Unkräuter wie z.B. Amaranthus, Datura und Ipomoea und in der Verträglichkeit z. B. in Soja gegenüber der Vergleichsverbindung (A) zeigt in diesem Test z.B. die Verbindung gemäß Herstellungsbeispiel (1).

## Ansprüche

1. Triazolo-pyrimidin-2-sulfonamide der Formel (I)

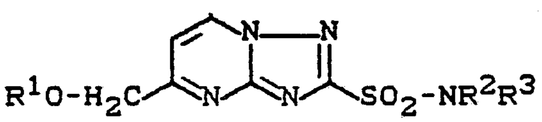

(I)

in welcher
$R^1$ für Alkyl steht,
$R^2$ für Wasserstoff, Alkyl, Alkylcarbonyl, Alkoxycarbonyl, Alkylsulfonyl, Alkenyl, Alkinyl oder für gegebenenfalls substituiertes Aralkyl steht und
$R^3$ für gegebenenfalls substituiertes Aryl steht.

2. Triazolo-pyrimidin-2-sulfonamide der Formel (I) gemäß Anspruch 1, in welcher
$R^1$ für Alkyl mit 1 bis 6 Kohlenstoffatomen steht,
$R^2$ für Wasserstoff, für Alkyl, Alkylcarbonyl, Alkoxycarbonyl und Alkylsulfonyl mit jeweils 1 bis 6 Kohlenstoffatomen im Alkylteil, für Alkenyl und Alkinyl mit jeweils 3 bis 8 Kohlenstoffatomen oder für

gegebenenfalls einfach oder mehrfach, gleich oder verschieden im Arylteil substituiertes Aralkyl mit 6 bis 10 Kohlenstoffatomen im Arylteil und 1 bis 4 Kohlenstoffatomen im Alkylteil steht, wobei als Arylsubstituenten in Frage kommen:

Fluor, Chlor, Brom oder Iod, Cyano, Nitro, Hydroxy, $C_1$-$C_6$-Alkyl, $C_1$-$C_6$-Alkoxy, $C_1$-$C_6$-Alkylthio, Halogen-$C_1$-$C_6$-alkyl, Halogen-$C_1$-$C_6$-alkoxy oder Halogen-$C_1$-$C_6$-alkylthio und

$R^3$ für gegebenenfalls einfach bis mehrfach, gleich oder verschieden substituiertes Aryl mit 6 bis 10 Kohlenstoffatomen steht, wobei als Arylsubstituenten in Frage kommen:

Fluor, Chlor, Brom oder Iod; Nitro; Cyano; Hydroxy; $C_1$-$C_6$-Alkyl; $C_1$-$C_6$-Alkoxy, $C_1$-$C_6$-Alkylthio; Halogen-$C_1$-$C_6$-alkyl; Halogen-$C_1$-$C_6$-alkoxy, Halogen-$C_1$-$C_6$-alkylthio; $C_1$-$C_6$-Alkylcarbonyl; $C_1$-$C_6$-Alkylsulfinyl; $C_1$-$C_6$-Alkylsulfonyl; Halogen-$C_1$-$C_6$-alkylsulfinyl; Halogen-$C_1$-$C_6$-alkylsulfonyl; Phenyl; Phenoxy; Phenylcarbonyl; Hydroxycarbonyl, $C_1$-$C_6$-Alkoxycarbonyl; $C_3$-$C_6$-Alkenyloxycarbonyl; $C_1$-$C_4$-Alkoxy-$C_1$-$C_6$-alkoxycarbonyl; Hydroximino oder $C_1$-$C_6$-Alkoximino.

3. Triazolo-pyrimidin-2-sulfonamide der Formel (I) gemäß Anspruch 1, in welcher

$R^1$ für Alkyl mit 1 bis 4 Kohlenstoffatomen steht,

$R^2$ für Wasserstoff, $C_1$-$C_4$-Alkyl, $C_1$-$C_4$-Alkylcarbonyl, $C_1$-$C_4$-Alkoxycarbonyl, $C_1$-$C_4$-Alkylsulfonyl, $C_3$-$C_6$-Alkenyl, $C_3$-$C_6$-Alkinyl und für gegebenenfalls einfach bis dreifach, gleich oder verschieden im Phenylteil substituiertes Phenyl-$C_1$-$C_4$-alkyl steht, wobei als Phenylsubstituenten in Frage kommen:

Fluor, Chlor, Brom, Iod, Hydroxy, Cyano, Nitro, Methyl, Ethyl, n-Propyl, i-Propyl, n-Butyl, i-Butyl, sec.-Butyl, tert.-Butyl, Methoxy, Ethoxy, n-Propoxy, i-Propoxy, n-Butoxy, i-Butoxy, sec.-Butoxy tert.-Butoxy, Methylthio, Ethylthio, n-Propylthio, i-Propylthio, n-Butylthio, i-Butylthio, sec.-Butylthio, tert.-Butylthio, Trifluormethyl, Trifluormethoxy oder Trifluormethylthio und

$R^3$ für gegebenenfalls einfach bis dreifach, gleich oder verschieden substituiertes Phenyl steht, wobei als Phenylsubstituenten in Frage kommen:

Fluor, Chlor, Brom Iod, Nitro, Cyano, Hydroxy, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy, Halogen-$C_1$-$C_4$-alkylthio, $C_1$-$C_4$-Alkylcarbonyl, $C_1$-$C_4$-Alkylsulfinyl, $C_1$-$C_4$-Alkylsulfonyl, Halogen-$C_1$-$C_4$-alkylsulfinyl, Halogen-$C_1$-$C_4$-alkylsulfonyl, Phenyl, Phenoxy, Phenylcarbonyl, Hydroxycarbonyl, $C_1$-$C_4$-Alkoxycarbonyl, $C_3$-$C_6$-Alkenyloxycarbonyl, $C_1$-$C_2$-Alkoxy-$C_1$-$C_4$-alkoxycarbonyl, Hydroximino oder $C_1$-$C_4$-Alkoximino.

4. Verfahren zur Herstellung von Triazolo-pyrimidin-2-sulfonamiden der Formel (I)

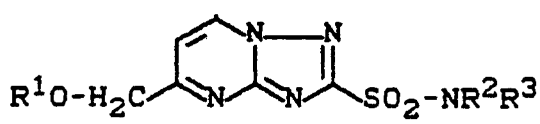

(I)

in welcher

$R^1$ für Alkyl steht,

$R^2$ für Wasserstoff, Alkyl, Alkylcarbonyl, Alkoxycarbonyl, Alkylsulfonyl, Alkenyl, Alkinyl oder für gegebenenfalls substituiertes Aralkyl steht und

$R^3$ für gegebenenfalls substituiertes Aryl steht,

dadurch gekennzeichnet, daß man Triazolo-pyrimidin-sulfonsäurechloride der Formel (II)

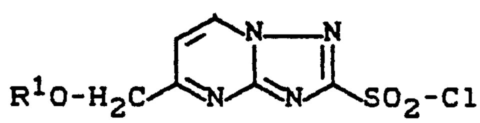

(II)

in welcher

$R^1$ die oben angegebene Bedeutung hat,

mit Aminen der Formel (III)

$R^3NH_2$ (III)

in welcher

$R^3$ die oben angegebene Bedeutung hat,

gegebenenfalls in Gegenwart von Säureakzeptoren, gegebenenfalls in Gegenwart von Katalysatoren und gegebenenfalls in Gegenwart von Verdünnungsmitteln umsetzt und gegebenenfalls anschließend die dabei entstehenden Triazolo-pyrimidin-2-sulfonamide der Formel (Ia)

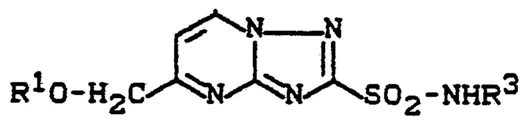

(Ia)

in welcher

$R^1$ und $R^3$ die oben angegebenen Bedeutung haben,

mit Verbindungen der Formel (IV)

$R^4W$ (IV)

in welcher

$R^4$ die gleiche Bedeutung wie $R^2$ hat, jedoch nicht für Wasserstoff steht und

W für eine nucleophile Abgangsgruppe steht,

gegebenenfalls in Gegenwart von Säureakzeptoren und gegebenenfalls in Gegenwart von Verdünnungsmitteln umsetzt.

5. Herbizide Mittel, gekennzeichnet durch einen Gehalt an mindestens einem Triazolo-pyrimidin-2-sulfonamid der Formel (I) gemäß Ansprüchen 1 bis 4.

6. Verfahren zur Bekämpfung von Unkräutern, dadurch gekennzeichnet, daß man Triazolo-pyrimidin-2-sulfonamide der Formel (I) gemäß Ansprüchen 1 bis 4 auf die Unkräuter und/oder ihren Lebensraum einwirken läßt.

7. Verwendung von Triazolo-pyrimidin-2-sulfonamiden der Formel (I) gemäß den Ansprüchen 1 bis 4 zur Bekämpfung von Unkräutern.

8. Verfahren zur Herstellung von herbixiden Mitteln, dadurch gekennzeichnet, daß man Triazolo-pyrimidin-2-sulfonamide der Formel (I) gemäß den Ansprüchen 1 bis 4 mit Streckmitteln und/oder oberflächenaktiven Substanzen vermischt.

9. Triazolo-pyrimidin-sulfonsäurechloride der Formel (II)

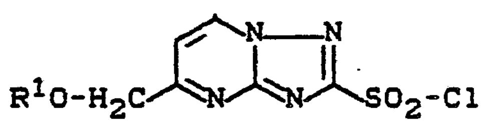

(II)

in welcher

$R^1$ für Alkyl steht.

10. Verbindungen der Formel (X)

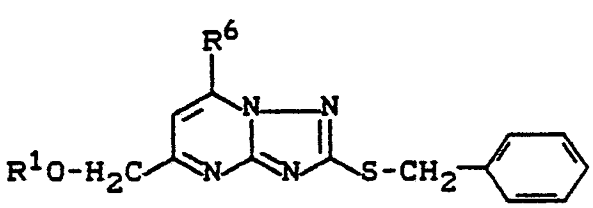

(X)

in welcher

$R^1$ für Alkyl steht und

$R^6$ für Wasserstoff, Chlor oder Hydroxy steht.

Europäisches Patentamt

# EUROPÄISCHER RECHERCHENBERICHT

Nummer der Anmeldung

EP 87111105.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| D,A | EP - A2 - 0 142 152 (THE DOW CHEMICAL COMPANY)<br>* Ansprüche 1,43-47,54,67; Seiten 1-6,20,21,24-26,34, 35 * | 1-10 | C 07 D 487/04<br>A 01 N 43/90//<br>(C 07 D 487/04<br>C 07 D 249:00<br>C 07 D 239:00) |
| A | DE - A - 1 620 694 (VEB DEUTSCHES HYDRIERWERK RODLEBEN)<br>* Anspruch 1; Seite 4 * | 1-3,9, 10 | |
| A | GB - A - 951 652 (IMPERIAL CHEMICAL INDUSTRIES, W.BROADBENT et al.)<br>* Ansprüche 1,5,6 * | 1-4,9, 10 | |
| A | DE - A - 2 327 133 (ICN PHARMACEUTICAL, INC.)<br>* Anspruch 1 * | 10 | RECHERCHIERTE SACHGEBIETE (Int. Cl 4) |
| A | EP - A1 - 0 183 848 (YOSHITOMI PHARMACEUTICAL INDUSTRIES, LTD.)<br>* Anspruch 1 * | 1-3,9, 10 | C 07 D 487/00 |

Der vorliegende Recherchenbericht wurde für alle Patentansprüche erstellt.

| Recherchenort | Abschlußdatum der Recherche | Prüfer |
|---|---|---|
| WIEN | 13-10-1987 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument